# EUROPEAN PATENT APPLICATION

(11) **EP 2 703 383 A1**
(43) Date of publication of application: **05.03.2014**
(21) Application number: 13179063.6
(22) Date of filing: 02.08.2013
(51) Int. Cl.: C07C 233/18, C07C 231/02, C07C 231/10, C07C 41/26, C07C 303/28, C07C 213/08

(54) **Process for the preparation of agomelatine**

(30) Priority: 27.08.2012 IT MI20121444
(71) Applicant: Procos S.p.A., 28062 Cameri NO (IT)
(72) Inventor: Barozza, Alessandro, 20020 NOSATE (MI) (IT); Veronese, Martino, 20022 CASTANO PRIMO (MI) (IT); Roletto, Jacopo, 10135 TORINO (IT); Paissoni, Paolo, 10040 DRUENTO (TO) (IT)
(74) Representative: Minoja, Fabrizio

(57) **Abstract**

Disclosed is a process for the synthesis of Agomelatine (1), N-(2-(7-methoxynaphthalen-1-yl)ethyl)acetamide from ethyl 2-(7-methoxynaphthalen-1-yl)acetate (4).

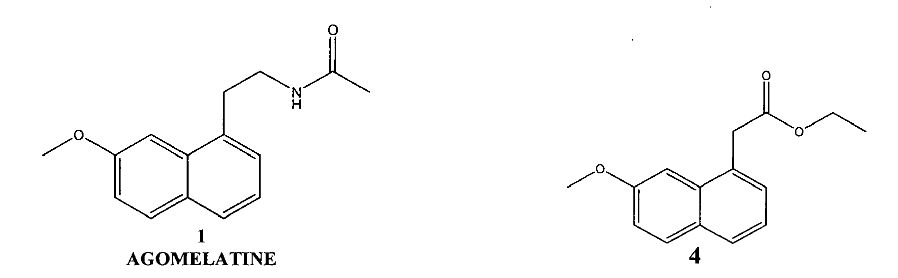

Said synthesis method is particularly advantageous compared with known procedures because it uses low-cost reagents under mild reaction conditions and allows the isolation of a product with excellent yields and quality.

## Description

### FIELD OF THE INVENTION

The present invention relates to a process for the preparation of Agomelatine from ethyl 2-(7-methoxynaphthalen-1-yl)acetate via the preparation of the novel intermediate N-formyl-N-(2-(7-methoxynaphthalen-1-yl)ethyl)formamide.

### BACKGROUND OF THE INVENTION

A number of processes are known for the synthesis of Agomelatine, an antidepressant and anxiolytic indicated for the treatment of major depressive disorder.

EP447285 discloses the synthesis of Agomelatine according to the scheme below:

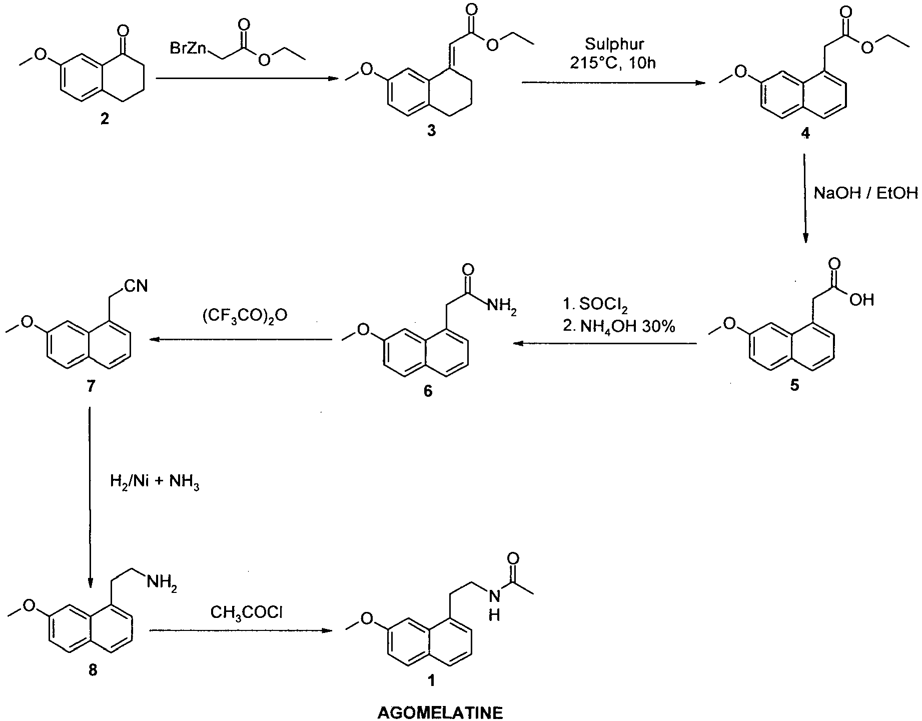

Said process is particularly disadvantageous for various reasons, including the following: the aromatisation step of intermediate 3 is conducted with sulphur at temperatures well above 200°C. These high temperatures, as well as not being particularly suitable for industrial-scale production because they require particular heating plants that use specific control units and diathermic oils, cause the degradation of organic substrates with the formation of impurities, leading to poor process yields. The synthesis of intermediate 7 by dehydration of the amide of intermediate 6 is performed with trifluoroacetic anhydride, the very high cost of which makes the process economically inefficient.

The reduction of the nitrile group of intermediate 7 to amine is particularly disadvantageous due to the formation of secondary and tertiary amines, which are difficult to eliminate. The formation of said impurities is well known (Hydrogenation methods, author Paul N. Rylander), and is only limited by the use of a very strong molar excess of ammonia compared with the product of reduction. However, such an excess of ammonia involves complicated management of its recycling, or its disposal by quenching with acids, which produces considerable amounts of inorganic salts as by-products.

WO200577887, WO200577888, WO200577889, WO200577890, WO2010015744, CN 101735091, CN 101792400, WO2011006387, CN102146046, CN102001960, CN102206170, CN102367228, CN102408350 and CN102531942 disclose the synthesis of Agomelatine according to the following scheme:

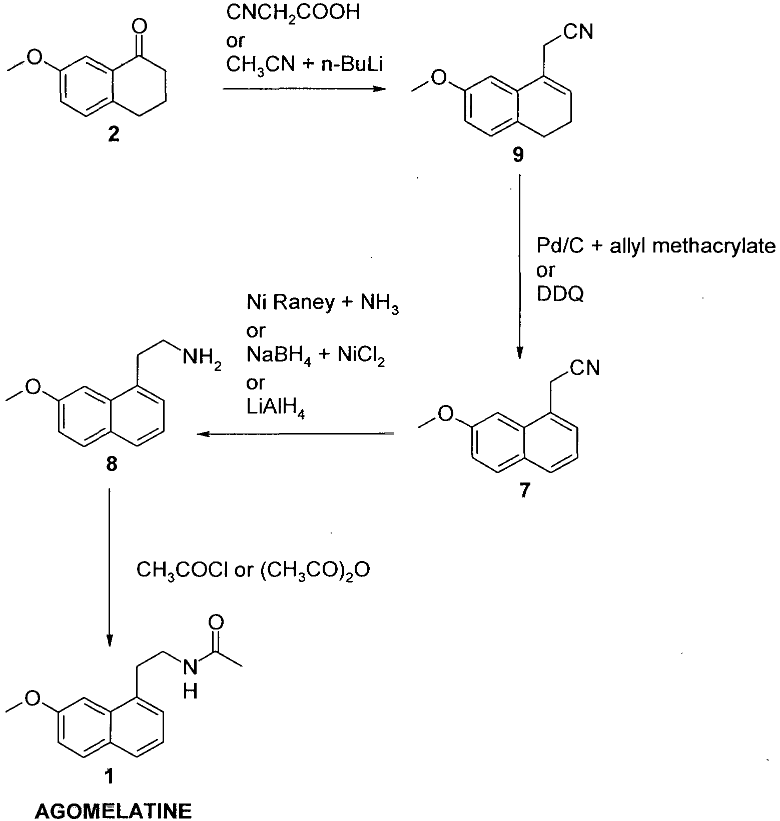

The synthesis of intermediate 7 with the nitrile group is performed by coupling the carbonyl of intermediate 2 with cyanoacetic acid or acetonitrile. Especially when acetonitrile is used as the source of the CN group, the reaction requires the presence of very strong bases such as n-BuLi and very low temperatures (-78°C), making the process unsuitable for application on an industrial scale. The aromatisation of intermediate 9 is performed with oxidising agents such as DDQ, a dangerous reagent which releases hydrochloric acid and hydrocyanic acid when in contact with water, or is highly toxic when in contact with metal catalysts such as Pd/C and allyl derivatives such as allyl methacrylate. The reduction of the nitrile group of intermediate 7 is described in the above-mentioned patent applications with different procedures, such as high-pressure hydrogenation in the presence of Raney Ni and a strong excess of ammonia, or with very aggressive reducing agents such as LiAlH₄ or other reducing agents which are milder but need metal catalysts such as NiCl₂. It is clear from all these methods that the reduction of the nitrile group to primary amine is particularly disadvantageous compared, for example, with the reduction of an ester group, in terms of the quality of the product, which can be contaminated by heavy metals, and the industrial applicability of the process, due to the drastic reaction conditions.

WO2012046253 and WO2011153939 describe some variations on the synthesis of Agomelatine similar to the synthesis route just described, involving coupling of the carbonyl of intermediate 2 with cyanoacetic acid or acetonitrile to synthesise common intermediate 10, wherein the OH group has not yet undergone the dehydration reaction to give the corresponding alkene.

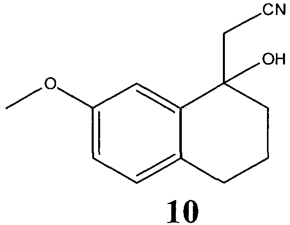

One of the main disadvantages of these two methods, as well as those already cited of reducing the nitrile group to amine, is that aromatisation is performed as the last step, which involves greater formation of impurities and lower yields.

WO2011154140 discloses a process for the preparation of Agomelatine which comprises synthesis of the nitrile intermediate by Wittig reaction between 7-methoxytetralone (intermediate 2) and diethyl cyanomethyl phosphonate, subsequent reduction to primary amine, acetylation and aromatisation. This process also has the same disadvantages of reduction of the nitrile group and aromatisation as the last synthesis step.

WO2012070025, CN101709036 and CN101486665 describe a similar process for the synthesis of Agomelatine to the one reported in EP447285, but with the difference that intermediate ester 4 is converted to intermediate 6 by ammonolysis, and amide 6 is directly reduced to the primary amine, as shown in the scheme below:

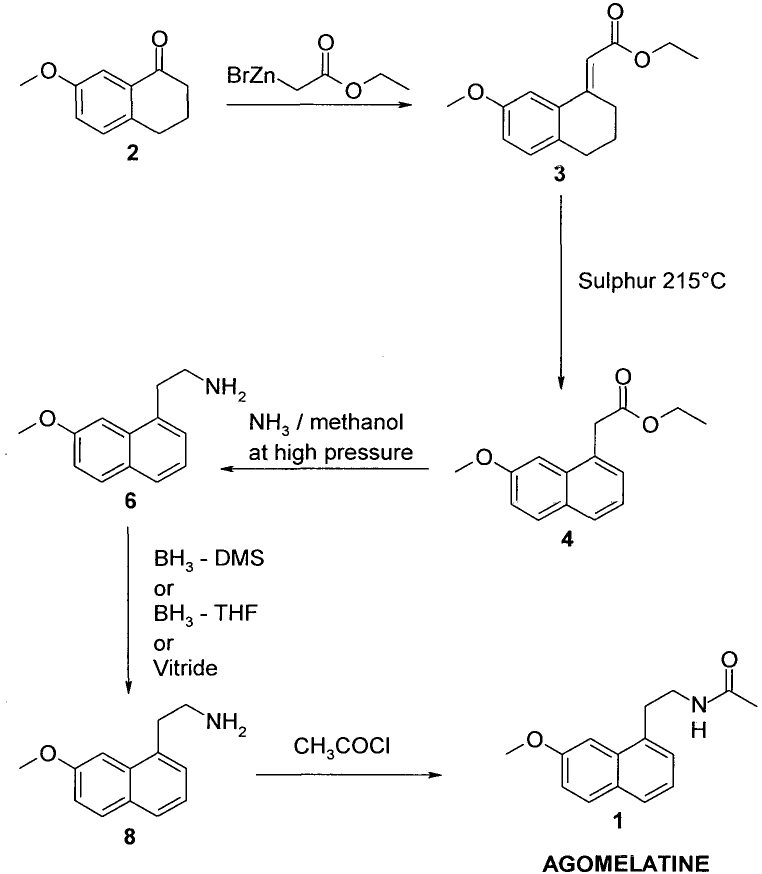

The reduction of the amide group requires, unlike the reduction of an ester group, for example, strongly reducing and very expensive reagents like BH₃ or Vitride.

CN10197387 reports the synthesis of Agomelatine via synthesis of acyl azide intermediate 11 with the use of diphenylphosphoryl azide, which is highly toxic and hazardous.

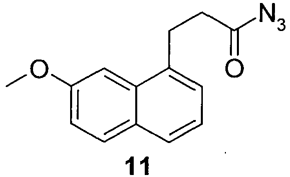

WO2010012208 discloses the synthesis of Agomelatine starting from alcoholic intermediate 12 by inserting a leaving group, synthesis of intermediate 14 with potassium phthalimide, hydrolysis to amine intermediate 8, and final acetylation. Basically, the approach of this process is based on the well-known Gabriel synthesis of primary amines as indicated in the scheme below:

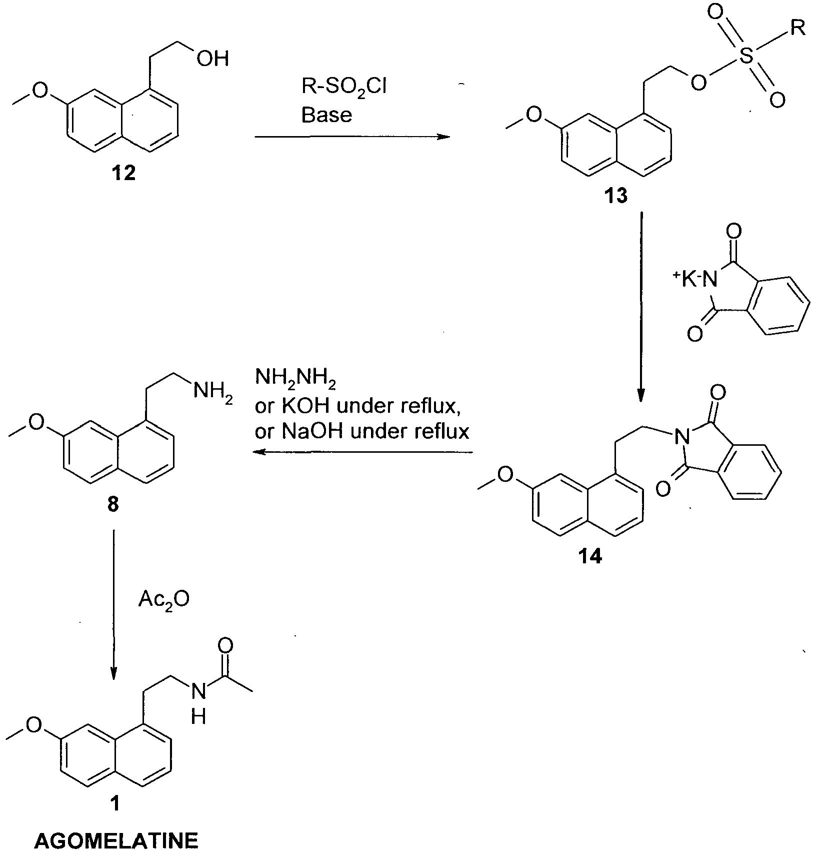

The synthesis of the primary amine (intermediate 8) through the use of Gabriel synthesis via potassium phthalimide is characterised by very low atom economy, as demonstrated by the need to dispose of large amounts of by-products such as phthalic acid or 2,3-dihydrophthalazine-1,4-dione (in the case of deprotection with hydrazine) in an equimolar quantity compared with the starting substrate. Moreover, due to its low atom economy, the synthesis of intermediate 14 is performed with ratios between solvent volume and substrate exceeding 10, thus considerably reducing the productivity of the process. Finally, the solvents used, such as acetonitrile and N,N-dimethylformamide, are characterised by high toxicity.

Other disadvantages include the drastic conditions for hydrolysis of intermediate 14, which use hydrazine (a carcinogen) or potassium or sodium hydroxide under reflux.

Finally, the process of WO2010012208 starts from intermediate 12 which, according to the literature (EP745586), is synthesised as described in the following scheme:

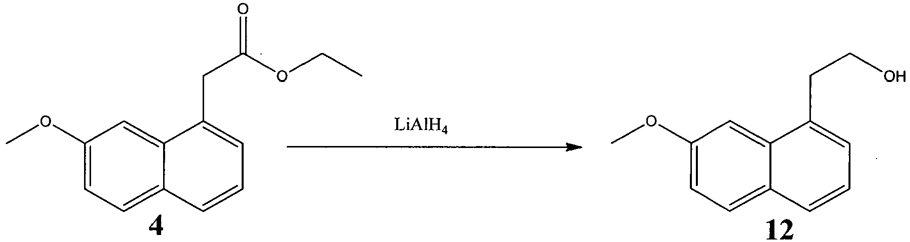

This procedure uses lithium aluminium hydride, which is difficult to apply industrially due to its very high reactivity with the moisture in the air, with which it releases easily inflammable hydrogen.

WO2010015745 discloses the synthesis of Agomelatine using catalysed Pd cross-coupling reactions that allow the synthesis of intermediate 17 from intermediate 16, prepared from 7-methoxy-1-naphthol (intermediate 15).

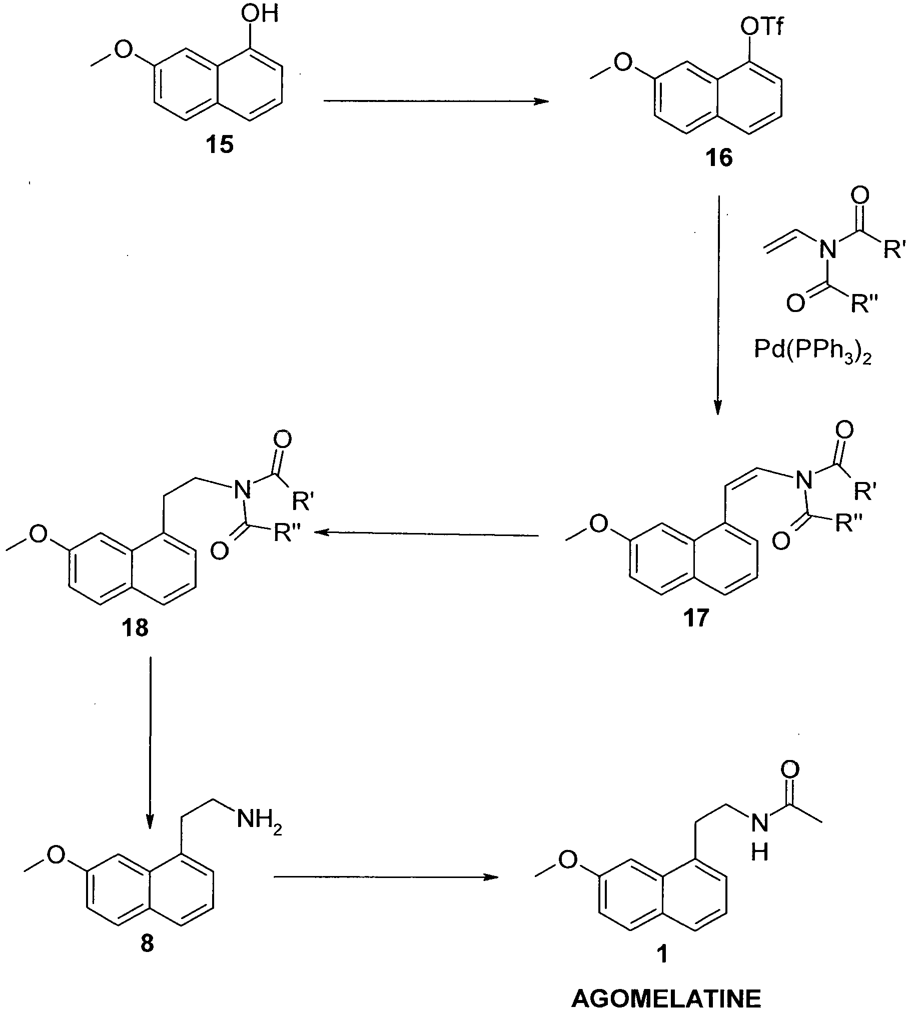

The main drawbacks of this procedure are that intermediate 15 is not commercially available (it can be obtained, for example, from 7-methoxytetralone (intermediate 2) with at least two synthesis steps), and that the catalysts used in the homogenous phase are very expensive and very difficult to recycle.

WO2009053545 discloses the synthesis of Agomelatine starting with intermediate 19 according to the scheme below:

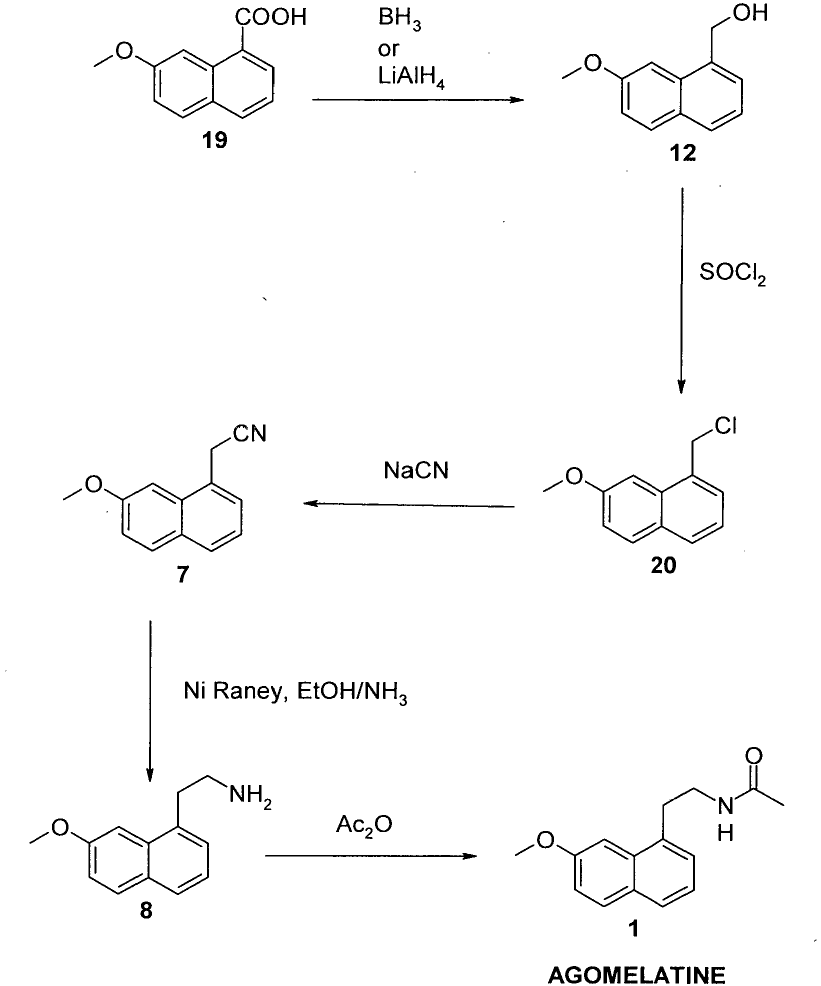

The main drawbacks of this method are as follows: intermediate 19 is not commercially available and its preparation requires numerous synthesis steps, the reduction of the carboxyl group to an alcohol group requires the use of strongly reducing reagents, and finally its preparation presents all the problems already listed relating to the reduction of nitriles to primary amines. Sodium cyanide is also used, the management of which on an industrial scale involves considerable safety problems.

WO2010015746 describes the synthesis of Agomelatine starting from intermediate 21 according to the scheme below:

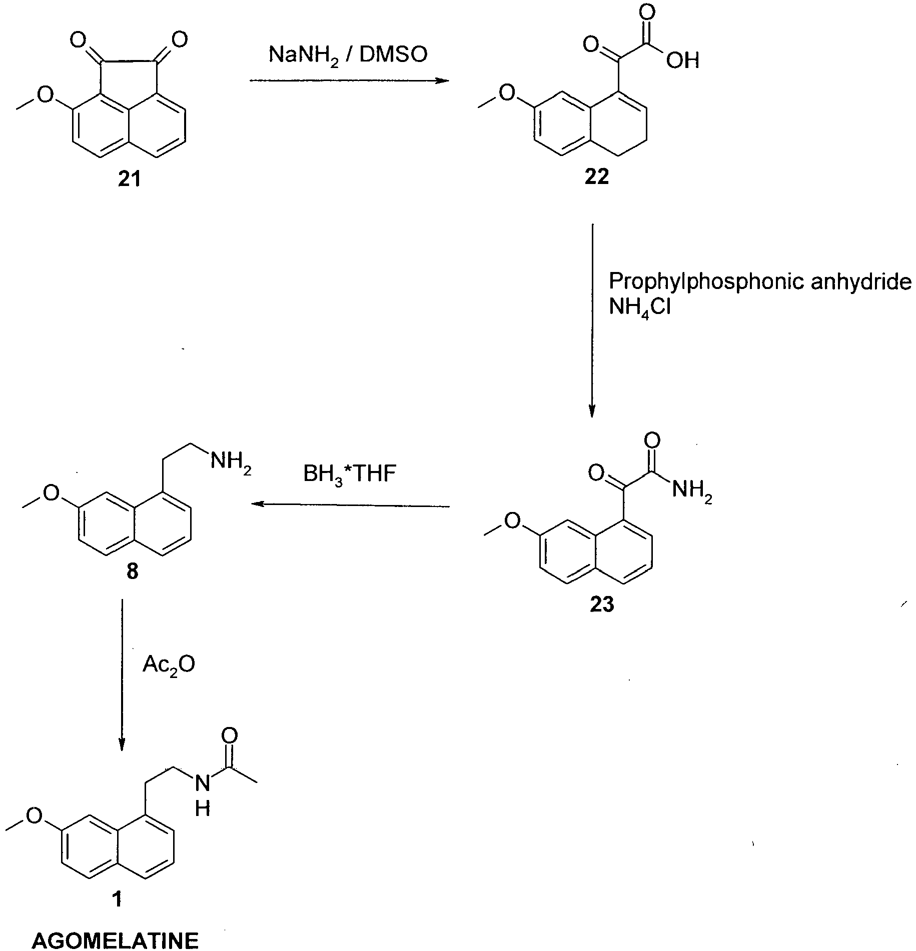

The main drawbacks of this method are as follows: intermediate 21 is not commercially available, and its preparation requires numerous synthesis steps. The reduction of the 2-oxoacetamide group also requires the use of strongly reducing, very expensive reagents like BH₃.

The known processes for the synthesis of Agomelatine are therefore unsatisfactory. There is accordingly a need for a process that combines characteristics of economic efficiency with scalability for industrial production of this active ingredient.

### DESCRIPTION OF THE INVENTION

A process has now been found for the preparation of Agomelatine from ethyl 2-(7-methoxynaphthalen-1-yl)acetate **4** via the preparation of the novel intermediate N-formyl-N-(2-(7-methoxynaphthalen-1-yl)ethyl)formamide **27**, according to the scheme below:

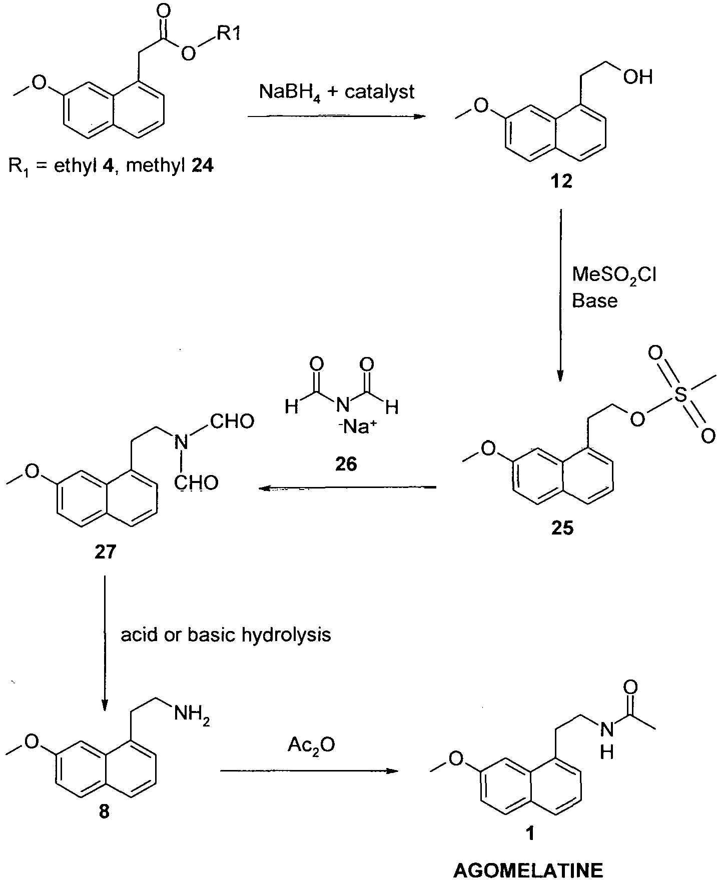

The process according to the invention comprises:
a) reduction of 2-(7-methoxynaphthalen-1-yl)acetic acid ethyl ester 4 or methyl ester 24 with sodium borohydride in the presence of a catalyst;
b) activation of the hydroxyl of compound 12 by inserting a leaving group;
c) nucleophilic substitution by diformylamide sodium salt 26;
d) hydrolysis of the N,N-diformyl intermediate;
e) acetylation with acetic anhydride to produce Agomelatine 1.

2-(7-Methoxynaphthalen-1-yl)acetic acid ethyl ester 4, which can be obtained commercially or conveniently synthesised from 7-methoxytetralone according to known methods, is preferred as the starting material.

The reduction reaction of the ester of formula 4 to give the compound of formula 12 is carried out with a solvent selected from alcohols such as methanol and ethanol, aliphatic or cyclic ethers, or mixtures of water and alcohols. According to a preferred procedure, the solvent is THF which, when the starting product has been added and dissolved, is added with 1 to 4 moles of sodium borohydride with respect to the starting ester, preferably 2 moles, followed by a neutral, acid or Lewis acid catalyst such as boron trifluoride, acetic acid, ethanol, calcium or zinc chloride, preferably 1 to 4 moles of boron tetrafluoride etherate with respect to the starting ester, preferably 1-2 moles, the temperature being maintained between 10 and 60°C. At the end of the reaction, after the addition of an extraction solvent and acid washes, a solution is obtained which is evaporated to dryness thus affording compound 4 in an almost quantitative yield. The manufacturing process of the compound of formula 12, involving reduction with sodium borohydride of the 2-(7-methoxynaphthalen-1-yl)acetic acid methyl or ethyl esters, is a further object of the invention.

Intermediate of formula 25 is obtained by reacting compound of formula 12, dissolved in a suitable aprotic solvent such as toluene, dichloromethane, pyridine or ethyl acetate, or a ketone such as MIBK, preferably toluene, with 1-4 equivalents, preferably 1.25, of mesyl chloride in the presence of 1-4 equivalents, preferably 1.5, of a suitable tertiary aliphatic, aromatic or substituted aromatic base such as triethylamine, diisopropylethylamine, pyridine or lutidine, preferably triethylamine, at a temperature between -10°C and 40°C. After suitable aqueous and aqueous acid washes, the solution is evaporated to dryness to provide compound 25 in an almost quantitative yield and high chromatographic purity.

Intermediate of formula 27 is synthesised from compound 25 and dissolved in a suitable aprotic polar solvent such acetonitrile, DMF or DMSO, preferably DMSO, usually employing 1-3 volumes of solvent with respect to compound 25, by reaction with diformylamide sodium salt of formula 26. Diformylamide sodium salt 26 is commercially available, and can also be prepared *in situ* by known methods from formamide and sodium methylate. The reaction is conducted with 1 to 4 equivalents of 26, preferably 1.5, maintaining the temperature at between 25°C and 70°C.

Intermediate of formula 8 is obtained by simple hydrolysis of intermediate 27. The hydrolysis can be performed under acid conditions, for example using hydrochloric acid, or under basic conditions, using inorganic bases such as NaOH and KOH. According to a preferred procedure, the hydrolysis is performed with 2-5 NaOH equivalents in a mixture of water and alcohols at a temperature between 35°C and the reflux temperature, preferably 40-50°C.

The synthesis of Agomelatine from intermediate 8 is performed with the use of acetic anhydride or acetyl chloride, according to known methods.

The Agomelatine obtained by the process according to the invention can be crystallised by known methods to obtain a product of sufficient purity and quality suitable for pharmaceutical use.

The advantages of the process of the invention are as follows:
- reduction of intermediate ester 4 and 24 with sodium borohydride (a reagent which is easily usable and very cheap) under mild reaction conditions and in high yields;
- use of the novel compound 27 which allows the alcohol group to be converted to amine, via the mesyl derivative with no need for isolation, with considerable atom economy and reduced production of reaction by-products compared with the procedures described in the literature, using solvents of low toxicity (DMSO) and extremely mild reaction conditions;

- deprotection of the amino group under mild hydrolysis conditions;
- very high yields at each step;
- due to the almost quantitative yields, Agomelatine can be synthesised from (4) without isolating the intermediates;
- Agomelatine of high purity is obtained.

The invention will be described in greater detail in the examples below.

### Example 1 - Synthesis of 2-(7-methoxynaphthalen-1-yl)ethanol 12

150 ml of ethanol is loaded into a three-necked reaction flask, magnetically stirred and equipped with a temperature probe, and cooled to 5°C. When the temperature of 5°C has been reached, 11.3 g of powdered calcium chloride (0.102 mol) is added, followed by 8.2 g of sodium borohydride (0.217 moles). After a few minute stirring at 5°C, 10 g of 2-(7-methoxynaphthalen-1-yl)acetic acid ethyl ester 4 (41 mmols), dissolved in 100 ml of anhydrous THF, is added, the temperature being maintained under 10°C. At the end of the addition the temperature is increased to room temperature and maintained at that temperature for about 2 hours. 5 ml of water is added slowly at the end of the reaction. 40 ml of toluene and 20 ml of 30% HCl are then added. The phases are separated by re-extracting the aqueous phase with 20 ml of toluene. The combined organic phases are washed with 40 ml of water, and then concentrated under vacuum to dryness to obtain 8.0 g of 2-(7-methoxynaphthalen-1-yl)ethanol 12. Yield 97%. The intermediate obtained was identified by NMR analysis: ¹H NMR (400 MHz, DMSO-d6) δ 7.83 (d, 2H), 7.70 (m, 1H), 7.36 (m, 2H), 7.27 (m, 1H), 7.18 (m, 1H), 3.92 (s, 3H), 3.75 (t, 2H), 3.29 (s, 1H), 3.19 (m, 1H).

### Example 2 - Synthesis of 2-(7-methoxynaphthalen-1-yl)ethanol 12

19.89 g of 2-(7-methoxynaphthalen-1-yl)acetic acid ethyl ester 4 (81.4 mmols) is loaded into a three-necked reaction flask, magnetically stirred and equipped with a temperature probe, and dissolved in 70 ml of anhydrous THF. 4.09 g of sodium borohydride (108 mmols) is added and then, maintaining the temperature at between 15 and 20°C, a solution of 11.79 g of boron trifluoride etherate (83.1 mmols) is slowly dropped into 20 ml of anhydrous THF. At the end of the addition the temperature is increased to 45°C and maintained at that temperature for 3 hours. The mixture is then cooled to 25°C, and 10 ml of water is added slowly. 70 ml of toluene and 40 ml of 30% HCl are then added. The phases are separated by re-extracting the aqueous phase with 30 ml of toluene. The combined organic phases are washed with 80 ml of water, and then concentrated under vacuum to dryness to obtain 18.96 g of 2-(7-methoxynaphthalen-1-yl)ethanol 12. Yield 99%. The intermediate obtained was identified by NMR analysis: ¹H NMK (400 MHz, DMSO-d6) δ 7.83 (d, 2H), 7.70 (m, 1H), 7.36 (m, 2H), 7.27 (m, 1H), 7.18 (m, 1H), 3.92 (s, 3H), 3.75 (t, 2H), 3.29 (s, 1H), 3.19 (m, 1H).

### Example 3 - 2-(7-methoxynaphthalen-1-yl)ethyl methanesulphonate 25

85 ml of methylene chloride, 9.1 g of 2-(7-methoxynaphthalen-1-yl)ethanol 12 (45 mmols) and 6.4 g of methanesulphonyl chloride (56 mmols) are loaded into a three-necked reaction flask, magnetically stirred and equipped with a temperature probe. The temperature is maintained at under 15°C, and 6.9 g of triethylamine (68 mmols) is added dropwise. At the end of the addition, after a 1-hour wait the reaction is quenched by slowly adding 60 ml of 0.5% HCl. The phases are separated and the organic phase is concentrated to dryness at low pressure to provide 12.3 g of 2-(7-methoxynaphthalen-1-yl)ethyl methanesulphonate 25. Yield 98%. The intermediate obtained was identified by NMR analysis: ¹H NMR (400 MHz, CDCl₃) δ 7.80 (m, 1H), 7.74 (m, 1H), 7.38 (m, 1H), 7.34 (m, 1H), 7.31 (m, 1H), 4.56 (t, 2H), 3.99 (s, 3H), 3.53 (t, 2H), 2.86 (s, 3H).

### Example 4 - N-formyl-N-(2-(7-methoxynaphthalen-1-yl)ethyl)formamide 27

3.0 g of 2-(7-methoxynaphthalen-1-yl)ethyl methanesulphonate (10.7 mmols), 1.53 g of sodium diformylamide (16.1 mmols) and 4 ml of dimethyl sulphoxide are loaded into a three-necked reaction flask, magnetically stirred and equipped with a temperature probe. The mixture is heated to 50°C and maintained at 50°C for about three hours. At the end of the reaction the mixture is cooled to room temperature and 10 ml of water is added. The quenched reaction mixture is extracted three times with 10 ml of methylene chloride each time. The organic phase is washed three times with 20 ml of water, dried by azeotropic distillation and concentrated under vacuum to obtain a residue amounting to 2.48 g of N-formyl-N-(2-(7-methoxynaphthalen-1-yl)ethyl)formamide 27. Yield 90%. The intermediate obtained was identified by NMR analysis: ¹H NMR (400 MHz, CDCl₃) δ 8.83 (s, 2H), 7.68 (m, 1H), 7.61 (m, 1H), 7.54 (m, 1H), 7.29 (m, 1H), 7.19 (m, 1H), 7.09 (m, 1H), 3.98 (s, 3H), 3.90 (t, 2H), 3.17 (t, 2H).

### Example 5 - 2-(7-methoxynaphthalen-1-yl)ethanamine 8

The residue obtained from example 4 is dissolved in 15 g of methanol in a three-necked reaction flask, magnetically stirred and equipped with a temperature probe, and 2.24 g of NaOH (56 mmols) is added. The reaction mixture is heated to 40°C and maintained at that temperature for about 18 hours. At the end of the reaction the solvent is evaporated and 25 ml of water is added. The resulting mixture is extracted three times with 10 ml of toluene each time. The organic phase is washed with 20 ml of water and then dried by azeotropic distillation. A small portion of the solution obtained is concentrated to dryness to identify intermediate 2-(7-methoxynaphthalen-1-yl)ethanamine 8 by NMR analysis: ¹H NMR (400 MHz, CDCl₃) δ 7.79 (m, 1H), 7.70 (m, 1H), 7.31 (m, 3H), 7.19 (m, 1H), 4.72 (s, 2H), 3.97 (s, 3H), 3.21 (t, 2H), 3.14 (t, 2H).

### Example 6 - Agomelatine 1

1.6 g of acetic anhydride (15.6 mmols) is added to the toluene solution obtained from example 5 and loaded into a three-necked reaction flask, magnetically stirred and equipped with a temperature probe. The reaction mixture is heated to 40°C and maintained at that temperature for about 3 hours. At the end of the reaction, 20 ml of water is added, the phases are separated, and the washing is repeated twice more. The organic phase is evaporated to obtain 2.15 g of Agomelatine. 83% yield from the intermediate 2-(7-methoxynaphthalen-1-yl)ethyl methanesulphonate 25. The product obtained was identified by NMR analysis: ¹H NMR (400 MHz, CDCl₃) δ 7.78 (m, 1H), 7.71 (m, 1H), 7.49 (m, 1H), 7.30 (m, 2H), 7.19 (m, 1H), 5.66 (s, 1H), 4.02 (s, 3H), 3.65 (t, 2H), 3.28 (t, 2H), 1.98 (s, 3H).

### Example 7 - Agomelatine 1 from 2-(7-methoxynaphthalen-1-yl)acetic acid ethyl ester intermediate 4 without isolation of intermediates

250 g of 2-(7-methoxynaphthalen-1-yl)acetic acid ethyl ester 4 (1.02 moles) is loaded into a three-necked reaction flask, magnetically stirred and equipped with a temperature probe, and dissolved in 850 ml of anhydrous THF. 51.4 g of sodium borohydride (1.36 mmols) is added and then, maintaining the temperature at between 15 and 20°C, a solution of 150 g of boron trifluoride etherate (1.06 mmols) is slowly dropped into 250 ml of anhydrous THF. At the end of the addition the temperature is increased to 45°C, and that temperature is maintained for about 3 hours. The mixture is then cooled to 25°C, and 120 ml of water is added slowly. 850 ml of toluene and 500 ml of 30% HCl are then added. The phases are separated by re-extracting the aqueous phase with 350 ml of toluene. The combined organic phases are washed with 1 litre of water and dried by azeotropic distillation. 145 g of methanesulphonyl chloride (1.26 moles) is added to the resulting solution. The temperature is maintained at under 15°C, and 155 g of triethylamine (1.53 moles) is added dropwise. At the end of the addition, after a 1-hour wait, the reaction is quenched by slowly adding 1.4 litres of 0.5% HCl. The phases are separated and the organic phase is concentrated to obtain a mass weighing about 350 g. 400 ml of dimethyl sulphoxide is added to the mass, which is further concentrated until the toluene residue from the preceding reaction has been eliminated. 142 g of sodium diformylamide (1.49 moles) is added to the resulting mass. The mixture is heated to 50°C and maintained at 50°C for about three hours. At the end of the reaction the mixture is cooled to room temperature, and 900 ml of water is added. The quenched reaction mixture is extracted three times, with 900 ml of methylene chloride each time. The organic phase is washed three times with 1.8 litres of water, dried by azeotropic distillation and concentrated under vacuum until the methylene chloride has been eliminated. The residue obtained is dissolved in 1.3 litres of methanol, and 208 g of NaOH (5.2 moles) is added. The reaction mixture is heated to 40°C and maintained at that temperature for about 18 hours. At the end of the reaction the solvent is evaporated, and 2 litres of water are added. The resulting mixture is extracted with 2 litres of toluene. The organic phase is washed with 1.8 litres of water and then dried by azeotropic distillation. 150 g of acetic anhydride (1.47 moles) is added to the resulting toluene solution. The reaction mixture is heated to 40°C and maintained at that temperature for about 3 hours. At the end of the reaction, 1.8 litres of water are added, the phases are separated, and the washing is repeated twice more. The organic phase is evaporated, to obtain 200 g of Agomelatine. 80% yield from the intermediate 2-(7-methoxynaphthalen-1-yl)acetic acid 4 ethyl ester.

## Claims

1. A process for the preparation of Agomelatine which comprises:
a) reduction of 2-(7-methoxynaphthalen-1-yl)acetic acid methyl or ethyl ester with sodium borohydride in the presence of a catalyst;
b) activation of the hydroxyl of the compound obtained in a) by inserting a leaving group;
c) reaction of the compound obtained in b) with diformylamide sodium salt;
d) hydrolysis of the intermediate obtained in c);
e) acetylation with acetic anhydride to afford Agomelatine.

2. A process according to claim 1 wherein the starting material is 2-(7-methoxynaphthalen-1-yl)acetic acid ethyl ester.

3. A process according to claim 1 or 2 wherein the reduction with sodium borohydride is carried out in tetrahydrofuran in the presence of boron etherate.

4. A process according to any one of claims 1 to 3 wherein step b) is carried out by reaction with mesyl chloride in an aprotic solvent in the presence of a tertiary aliphatic, aromatic or substituted aromatic base.

5. A process according to any one of claims 1 to 4 wherein step c) is carried out in a polar aprotic solvent, preferably dimethylsulphoxide.

6. A process according to any one of claims 1 to 5 wherein the hydrolysis of step d) is carried out with 2-5 NaOH equivalents in a water-alcohol mixture at a temperature ranging between 35°C and the reflux temperature, preferably 40°C - 50°C.

7. A process for the preparation of 2-(7-methoxynaphthalen-1-yl)ethanol 12 by reduction of 2-(7-methoxynaphthalen-1-yl)acetic acid methyl or ethyl ester with sodium borohydride in the presence of catalysts.

8. A process according to claim 7 wherein the reduction is carried out in a solvent selected from alcohols, aliphatic or cyclic ethers, or water-alcohol mixtures.

9. A process according to claim 7 or 8 wherein the catalyst is selected from boron trifluoride, boron tetrafluoride etherate, acetic acid, ethanol, calcium and zinc chloride.

10. A process according to claim 9 wherein the solvent is tetrahydrofuran and the catalyst is boron tetrafluoride etherate.

11. N-Formyl-N-(2-(7-methoxynaphthalen-1-yl)ethyl)formamide of formula 27.
